# EUROPEAN PATENT SPECIFICATION

(11) **EP 0 923 515 B1**
(45) Date of publication and mention of the grant of the patent: **19.12.2001**
(21) Application number: 97928990.7
(22) Date of filing: 13.06.1997
(51) Int. Cl.: C07C 19/08, C01B 7/01

(54) **AZEOTROPE-LIKE COMPOSITIONS OF DIFLUOROMETHANE AND CHLORINE**
AZEOTROP-ÄHNLICHE ZUSAMMENSETZUNGEN VON DIFLUORMETHAN UND CHLOR
COMPOSITIONS AZEOTROPIQUES DE DIFLUOROMETHANE ET DE CHLORE

(30) Priority: 14.06.1996 US 20181 P; 03.06.1997 US 868399
(43) Date of publication of application: 23.06.1999
(73) Proprietor: Honeywell International Inc., Morristown, New Jersey 07962-2245 (US)
(72) Inventor: PHAM, Hang Thanh, Amherst, NY 14228 (US); SINGH, Rajiv, R., Getzville, NY 14068 (US); SMITH, Addison, M., Amherst, NY 14226 (US); WILSON, David, P., East Amherst, NY 14051 (US); THOMAS, Raymond, Hilton, Percival, Pendleton, NY 14094 (US); CERRI, Gustavo, Boontoon, NJ 07005 (US)
(74) Representative: Hucker, Charlotte Jane
(86) International application number: US9710450
(87) International publication number: WO9747574

(56) References cited:
- US-A- 3 694 362
- DATABASE CHEMABS CHEMICAL ABSTRACTS SERVICE, COLUMBUS, OHIO, US vol. 121, abstract no. 136839, VINOGRADOV, D. V. ET AL: "Study of azeotropic properties in systems formed by Khladon 21 and Khladon 22 with chlorine and hydrogen fluoride" XP002042207 & ZH. PRIKL. KHIM. (S.-PETERBURG) (1993), 66(10), 2389-92 CODEN: ZPKHAB;ISSN: 0044-4618, 1993,

## Description

The present invention relates to mixtures of difluoromethane ("HFC-32") and chlorine. More particularly, the invention provides azeotrope-like compositions of HFC-32 and chlorine.

### Background of the Invention

Chlorine is used by industry in a number of processes including in the production of chlorinated organic and inorganic chemicals. For example, methane may be chlorinated to produce methylene chloride, chloroform, or carbon tetrachloride. As another example, hydrogen and chlorine may be reacted to produce hydrochloric acid.

However, these chlorination reactions are highly exothermic reactions which require close temperature control. Additionally, in the manufacture of chlorine itself, cooling of the reactant and product vessels are required. Therefore, a need exists for a reactant that will permit these reactants to be carried out under less exothermic conditions.

US-A-3 694 362 discloses an azeotropic composition of dichlorodifluoromethane and chlorine, useful in the separation of dichlorodifluoromethane from mixtures with chlorine, and in refrigeration.

### Description of the Invention and Preferred Embodiments

This invention provides azeotrope-like compositions of HFC-32 and chlorine as defined in claim 1.

The preferred and most preferred compositions of the invention are set forth in Table 1.

**Table 1**

| Components | Preferred (wt%) | Most Preferred (wt %) |
|---|---|---|
| HFC-32 | 50-95 | 60-90 |
| Chlorine | 5-50 | 10-40 |

In a particularly preferred embodiment, the composition of the invention boils at about -55° ± about 1° C at about atmospheric pressure (745 mm Hg) In a still more particularly preferred embodiment, the composition of the invention comprises about 65 ± about 5 weight percent HFC-32 and about 35 ± about 5 weight percent chlorine which composition boils at about -55° ± about 10° C

For purposes of this invention, azeotrope-like compositions are compositions that behave like azeotropic mixtures. From fundamental principles, the thermodynamic state of a fluid is defined by pressure, temperature, liquid composition, and vapor composition. An azeotropic mixture is a system of two or more components in which the liquid composition and vapor composition are equal at the state pressure and temperature In practice, this means that the components of an azeotropic mixture are constant boiling and cannot be separated during a phase change.

Azeotrope-like compositions behave like azeotropic mixtures, i.e., or are constant boiling or essentially constant boiling. In other words, for azeotrope-like compositions, the composition of the vapor formed during boiling or evaporation is identical, or substantially identical, to the original liquid composition. Thus, with boiling or evaporation, the liquid composition changes, if at all, only to a minimal or negligible extent. This is to be contrasted with non-azeotrope-like compositions in which, during boiling or evaporation, the liquid composition changes to a substantial degree. All compositions of the invention within the indicated ranges as well as certain compositions outside these ranges are azeotrope-like

The azeotrope-like compositions of the invention may include additional components that do not form new azeotropic or azeotrope-like systems, or additional components that are not in the first distillation cut The first distillation cut is the first cut taken after the distillation column displays steady state operation under total reflux conditions One way to determine whether the addition of a component forms a new azeotropic or azeotrope-like system so as to be outside of this invention is to distill a sample of the composition with the component under conditions that would be expected to separate a nonazeotropic mixture into its separate components. If the mixture containing the additional component is nonazeotropic or nonazeotrope-like, the additional component will fractionate from the azeotropic or azeotrope-like components. If the mixture is azeotrope-like, some finite amount of a first distillation cut will be obtained that contains all of the mixture components that is constant boiling or behaves as a single substance.

It follows from this that another characteristic of azeotrope-like compositions is that there is a range of compositions containing the same components in varying proportions that are azeotrope-like, or constant boiling All such compositions are intended to be covered by the terms "azeotrope-like" and "constant boiling". As an example, it is well known that at differing pressures, the composition of a given azeotrope will vary at least slightly as does the boiling point of the composition. Thus, an azeotrope of A and B represents a unique type of relationship, but with a variable composition depending on temperature and/or pressure It follows that, for azeotrope-like compositions, there is a range of compositions containing the same components in varying proportions that are azeotrope-like. All such compositions are intended to be covered by the term azeotrope-like as used herein.

The compositions of the invention have a lower boiling point than either of the compositions' components. One ordinarily skilled in the art will recognize that the compositions of the invention may offer superior refrigeration capacity when compared to either chlorine or HFC-32 alone Thus, in one embodiment of the invention, the azeotrope-like compositions of the invention may be used in a method for cooling. In a preferred embodiment, the compositions are used in method for cooling the reactant and product vessels in a process for producing chlorine

In yet another embodiment of the invention, the compositions may find utility in chlorination reactions of organic and inorganic starting materials The chlorination of these materials is highly exothermic and, by using the azeotrope-like compositions of the invention in place of chlorine in the chlorination reactions, the heat generated by the reactions may be lowered by a factor of from about 3 to about 6 depending on the HFC-32/chlorine ratio Any organic or inorganic materials that may be chlorinated may be chlorinated using the compositions of the invention. Because the chlorination reactions of such materials are well known in the art, the reaction conditions and amounts of starting materials and of the azeotrope-like composition of the invention to be used will be readily ascertainable by one ordinarily skilled in the art.

In still another embodiment, the compositions of the invention may find utility in fluorination reactions. Liquid phase processes for the production of HFC-32 using antimony catalysts and chlorine feeds are well known in the art. In one liquid phase process for preparing HFC-32, methylene chloride ("HCC-30") is fluorinated with hydrogen fluoride in the presence of a fluorination catalyst, such as an antimony halide catalyst. Chlorine is typically added to the reactor to reactivate the catalyst It is known that combinations of hydrofluorocarbons and chlorine pose a potential flammability risk. Thus, the use of a chlorine feed in the production of HFC-32 provides the potential for formation of a flammable mixture of HFC-32 and chlorine if chlorine is permitted to build up to a high enough concentration.

During the HFC-32 production process, at least one distillation column separates HFC-32 from unreacted starting materials, such as HCC-30 and hydrogen fluroide, and the reaction intermediate, monochloromonofluoromethane ("HCFC-31"). Because the boiling point of chlorine, -34.4°C (-30°F), is between that of HFC-32, -52.7°C (-61° F), and HCFC-31, -26.4°F (-15.6°F), chlorine may build up to a high concentration in the middle of the distillation column even if chlorine is fed at a very low rate. It has been discovered that the HFC-32/chlorine azeotrope-like composition may be used for purging chlorine, along with HFC-32, from the distillation column. By controlling the chlorine feed, the formation of a flammable HFC-32/chlorine mixture in the column may be minimized if not avoided. Therefore, in still another embodiment of the invention, a process for producing HFC-32 is provided in which the azeotrope-like composition of the invention is used to control the rate of feed of chlorine in order to minimize, or avoid, the formation of a flammable HFC-32/chlorine mixture

The components of the compositions of the invention are commercially available or may be produced by known methods. Preferably, the components are of sufficiently high purity so as to avoid the introduction of adverse influences on the properties, such as constant boiling, of the compositions

The following non-limiting examples serve to illustrate the invention

### Examples

### Example 1

An ebulliometer which consisted of a vacuum jacketed tube with a condenser on top was used. About 20.8 grams of difluoromethane was charged into the ebulliometer and chlorine was added in measured small increments. The temperature was measured using a platinum resistance thermometer From about 0 to about 32 weight percent chlorine, the boiling point of the composition changed by only about 4° C Therefore the composition behaves as a constant-boiling over this range.

### Example 2

Table 2 shows the vapor pressure measurement of HFC-32 and chlorine as a function of composition of chlorine (weight percent chlorine) at constant temperature of about 20° C. From this Table it may be observed that at this temperature, the compositions at which the vapor pressure maximum is approximately 31.7 weight percent chlorine and between approximately 26.2 to 36.7 weight percent chlorine. The data also show that the vapor pressure of mixtures of HFC-32 and chlorine is higher, at all indicated blend proportions, than HFC-32 and chlorine alone, i.e. as indicated in the first and last rows when chlorine is 0.0 weight percent and HFC-32 is at 100 0 weight percent as well as when HFC-32 is at 0.0 weight percent and chlorine is at 100 0 weight percent.

**Table 2**

| WEIGHT PERCENT CHLORINE | PRESSURE in 10⁴ Pa (PSIA) |
|---|---|
| 0.0 | 147.1 (213.4) |
| 65 | 152.7 (221.5) |
| 9.8 | 154.9 (224.7) |
| 15.8 | 158.1 (229.3) |
| 20.6 | 159.8 (231.8) |
| 26.2 | 161.5 (234.3) |
| 31.7 | 161.5 (234.3) |
| 36.7 | 161.0 (233.5) |
| 41.7 | 160.4 (232.6) |
| 48.9 | 158.3 (229.7) |
| 100.0 | 69.43 (100.7) |

## Claims

1. An azeotrope-like composition comprising from 40 to 99 weight percent difluoromethane and from 1 to 60 weight percent chlorine, which composition boils at a temperature of 20° ± 4°C at about 161 x 10⁴ Pa (234 psia).

2. The composition of claim 1 wherein the composition boils at 20° ± 1.0°C at about 161 x 10⁴ Pa (234 psia).

3. The composition of claim 1 comprising from 50 to 95 weight percent difluoromethane and from 5 to 50 weight percent chlorine.

4. The composition of claim 3 comprising from 60 to 90 weight percent difluoromethane and from 10 to 40 weight percent chlorine.

5. The composition of claim 1 comprising 32 ± 5 weight percent chlorine and 68 ± 5 weight percent difluoromethane.

6. The composition of claim 1, which boils at -55° ± 1.0°C at about 993 x 10² Pa (745 mm Hg).

7. The composition of claim 6 comprising 35 ± 5 weight percent chlorine and 65 ± 5 weight percent difluoromethane.

## Patentansprüche

1. Azeotropartige Zusammensetzung, die 40 bis 99 Gewichtsprozent Difluormethan und 1 bis 60 Gewichtsprozent Chlor enthält und bei etwa 161 x 10⁴ Pa (234 psi abs.) bei einer Temperatur von 20 ± 4°C siedet.

2. Zusammensetzung nach Anspruch 1, die bei etwa 161 x 10⁴ Pa (234 psi abs.) bei einer Temperatur von 20 ± 1,0°C siedet.

3. Zusammensetzung nach Anspruch 1, die 50 bis 95 Gewichtsprozent Difluormethan und 5 bis 50 Gewichtsprozent Chlor enthält.

4. Zusammensetzung nach Anspruch 3, die 60 bis 90 Gewichtsprozent Difluormethan und 10 bis 40 Gewichtsprozent Chlor enthält.

5. Zusammensetzung nach Anspruch 1, die 32 ± 5 Gewichtsprozent Chlor und 68 ± 5 Gewichtsprozent Difluormethan enthält.

6. Zusammensetzung nach Anspruch 1, die bei etwa 993 x 10² Pa (745 mm Hg) bei einer Temperatur von -55 ± 1,0°C siedet.

7. Zusammensetzung nach Anspruch 6, die 35 ± 5 Gewichtsprozent Chlor und 65 ± 5 Gewichtsprozent Difluormethan enthält.

## Revendications

1. Composition de type azéotrope comprenant de 40 à 99 pour cent en poids de difluorométhane et de 1 à 60 pour cent en poids de chlore, ladite composition bouillant à une température de 20° ± 4°C à environ 161 x 10⁴ Pa (234 psia).

2. Composition selon la revendication 1 dans laquelle la composition bout à 20° ± 1,0°C à environ 161 x 10⁴ Pa (234 psia).

3. Composition selon la revendication 1 comprenant de 50 à 95 pour cent en poids de difluorométhane et de 5 à 50 pour cent en poids de chlore.

4. Composition selon la revendication 3 comprenant de 60 à 90 pour cent en poids de difluorométhane et de 10 à 40 pour cent en poids de chlore.

5. Composition selon la revendication 1 comprenant 32 ± 5 pour cent en poids de chlore et 68 ± 5 pour cent en poids de difluorométhane.

6. Composition selon la revendication 1 qui bout à -55° ± 1,0°C à environ 993 x 10² Pa (745 mm de Hg).

7. Composition selon la revendication 6 comprenant 35 ± 5 pour cent en poids de chlore et 65 ± 5 pour cent en poids de difluorométhane.
